Europäisches Patentamt

European Patent Office

Office européen des brevets

(11) Publication number: **0 182 131 B1**

# EUROPEAN PATENT SPECIFICATION

(45) Date of publication of patent specification: **18.12.91** (51) Int. Cl.5: **A61K 49/02, A61K 43/00**

(21) Application number: **85113387.6**

(22) Date of filing: **22.10.85**

(54) The use of micellular particle for making compositions for targeting tumors in humans.

(30) Priority: **23.11.84 US 674201**

(43) Date of publication of application:
**28.05.86 Bulletin 86/22**

(45) Publication of the grant of the patent:
**18.12.91 Bulletin 91/51**

(84) Designated Contracting States:
**AT BE CH DE FR GB IT LI LU NL SE**

(56) References cited:
**EP-A- 0 028 917**
**US-A- 4 086 330**

(73) Proprietor: **VESTAR, INC.**
**650 Cliffside Drive**
**San Dimas California 91773(US)**

(72) Inventor: **Presant, Cary Arnet**
**2345 Cumberland Road**
**San Marino, Calif. 91108(US)**
Inventor: **Proffitt, Richard Thomas**
**11 North Altura Road**
**Arcadia, Calif. 91006(US)**

(74) Representative: **Eitle, Werner, Dipl.-Ing. et al**
**Hoffmann, Eitle & Partner Patentanwälte Ar-**
**abellastrasse 4**
**W-8000 München 81(DE)**

## Description

This invention relates to the use of micellular particle compositions for targeting tumors in humans. More particularly, the invention relates to micellular particle compositions comprising neutral and/or charged micellular particles containing a radiolabeled marker.

Before various abnormalities in a patient's body can be diagnosed and treated, it is often necessary to locate the abnormalities. This is particularly true of abnormalities such as malignant tumors since the treatment is often on a localized basis. Thus, the location of the malignant tumor must be identified so that therapy can be directed to such cancer cells for treatment.

Various attempts have been made over an extended number of years to identify specific locations, such as tumors, by simple techniques. For example, it would be desirable to identify the location of cancer cells by a simple method involving the localization of a particular chemical at the specific site. It would also be desirable to treat the cancer by introducing modified chemicals into the patient's body and having such chemicals move to specific locations to combat the cancer cells at such locations. In spite of such attempts, however, simple delivery systems for targeting tumors in humans do not exist as yet.

Placing a chemotherapeutic drug in the body orally, subcutaneously or intravenously can result in harm to the normal cells in the body which take up the drug and a worsening in the patient's condition, without achieving the desired reduction in tumor cell activity. In the past, this toxicity to normal cells in the patient's body has been a major disadvantage in the treatment of tumors with chemotherapeutic agents. The lack of efficacy of such chemotherapy is also attributable to the failure of the freely circulating drug to localize within tumor cells before it is excreted or taken up by other cells in the body.

Prior attempts to improve treatment of tumors by chemotherapeutic agents have included encapsulation of such agents within biodegradable phospholipid micellular particles in the form of vesicles or liposomes. Encapsulation is thought to reduce potential toxicity from the circulating drugs. Researchers have also sought to utilize such encapsulation to selectively target tumors within a body for delivery of chemotherapeutics.

The inability to provide a satisfactory particle targeting method is believed to be due to the nature of the solid tumors and their metastases which are located in extravascular tissues. Thus, to accomplish targeting of intravenously injected radiolabelled or chemotherapeutic particles to the tumor cells, the particles must leave the normal circulation by crossing the blood vessel membranes to enter the extravascular tissues. This movement is known as "extravasation". In addition the encapsulated agent must cross the tumor cell membrane. Normally, small substances such as small molecular weight proteins and membrane-soluble molecules can cross cell membranes by a process known as passive diffusion. However, passive diffusion will not allow sufficient accumulation of larger particles carrying drugs within cells to reach therapeutic levels. Additionally, cells can actively transport materials across the membrane by a process such as pinocytosis wherein extracellular particles are engulfed by the membrane and released inside the cell. Entry of encapsulating particles into individual cells may occur by pinocytosis.

Progress in targeting tumors with chemotherapeutic drugs has been hampered by the inability to accomplish and detect movement of drug carriers across blood vessel membranes. In the usual case, large structures such as drug encapsulating vesicles cannot escape from blood vessels such as capillaries, and thus remain in circulation.

An understanding of extravasation, however, requires an examination of the structure of the vascular morphology of a tumor. Various blood vessels are associated with tumors, in particular capillaries. It is now known that tumor capillaries may exhibit alterations in their structure, such as fenestrations, as a result of tumor cell growth patterns. H.I. Peterson, Vascular and Extravascular spaces in Tumors: Tumor Vascular Permeability, Chapter III, Tumor Blood Circulation, H.I. Peterson, Ed. (1979). Studies of tumor capillary permeability reveal morphologic variations in the capillaries which allow some substances to cross the capillary membrane. Such variations include defects in vascular endothelium from poor cell differentiation, or breaks in vascular walls as a result of invading tumor cells. H.I. Peterson, supra. Notwithstanding such knowledge of tumor vascular morphology, researchers such as Peterson have concluded that transport of large molecules or materials across the tumor capillary wall occurs as a result of passive diffusion and that "concentrations of active drugs sufficient for therapeutic effect are difficult to reach". H.I. Peterson, supra, at 83.

Prior to such morphologic studies, early reports suggested that vesicles might undergo transcapillary passage across the capillary membranes into tumor cells. G. Gregoriadis, Liposomes in Biological Systems, Gregoriadis, Ed., Ch 2, (1980); cf. also EP-A-28917. However, available data indicated that the vesicles were unstable in vivo and that the radiolabel may have leaked, thus apparently prompting the two alternative theories of (1) longer circulation of vesicles in the blood with release of drugs at a slower rate or (2) interaction of the liposomes with the capillary walls without crossing the wall surface, which would result in the appearance of drugs at the tumor sites, but without drugs within tumor cells. Id. Other researchers simply concluded that the vesicles do not penetrate vascular walls after intravenous administration. B. Ryman et al., Biol. Cell, Vol. 47, pp. 71-80 (1983); G. Poste, Biol. Cell, Vol. 47, pp. 19-38 (1983).

Thus, although the prior art has recognized that vesicles carrying radiolabel markers or therapeutic drugs must cross vascular barriers to reach tumor cells, the experience of the art has taught that intravenous administration is not effective to deliver the vesicles to extravascular tumor cells. In the aforesaid application, Serial No. 663,503, and parent application, Serial No. 363,593, "Method of Targeting a Specific Location in a Body", the disclosures of which are incorporated herein by reference, a method is provided for targeting tumors in vitro and in animals, specifically, mice. In the present invention, a method is provided for enhancing extravasation of radiolabelled particles to tumor cells within humans, for the identification of such tumor sites.

Subject matter of the present invention is the use of micellular particles of less than 200 nm comprising chemically pure phospholipid molecules, said particles having incorporated a radioactive agent, for the preparation of pharmaceutical compositions for the diagnosis, imaging or treatment of tumors or metastatic cancers according to claims 1 to 3.

Preferred embodiments of one or more of the subject matter of these claims are subject of claims 4 to 15.

According to the invention phospholipid micellular particles such as vesicles that are pure (more than 98% pure) neutral phospholipid molecules are incorporated into small (less than 200nm micelles) as a component of the external surface. The phospholipid molecules are radiolabeled to enhance the idendity and the diagnosis of the tumor at the specific site.

In-111 labelled vesicles were injected intravenously into 13 patients with diagnoses of terminal cancer. Following intravenous injections of up to 275 mg of lipid and 18,5 MBq (500 micro-curies) of In-111, vesicles were rapidly taken up by the liver and spleen and a median of 12.5% of the injected vesicles restained within circulation at 24 hours. In no patient did symptoms develop related to vesicles following administration. Twelve of the thirteen patients had tumors imaged by this scan.

As used herein, "micellular particle" and "micelles" refer to particles which result from aggregations of amphihilic molecules. In this invention preferred amphiphiles are biological lipids. Micelles are water-soluble aggregates of molecules with hydrophobic and hydrophilic portions (so-called amphiphilic molecules) which associate spontaneously. Such micelles can be in the form of small spheres, ellipsoids or long cylinders, and can also consist of bilayers with two parallel layers of amphiphilic molecules. Such bilayered micelles usually take the shape of spherical vesicles with an internal aqueous compartment. Useful compositions of these micelles include phospholipid molecules in the structure.

"Vesicle" refers to a micelle which is in a generally spherical form, often obtained from a lipid which forms a bilayered membrane and is referred to as a "liposome". Methods for forming these vesicles are, by now, well known in the art. Typically, they are prepared from a phospholipid, for example, distearoyl phosphatidylcholine, and may include other materials such as neutral lipids, for example, cholesterol, and also surface modifiers such as positively or negatively charged compounds.

The phospholipid molecules may constitute distearoyl phosphatidylcholine. The stability of the distearoyl phosphatidylcholine micelles may be enhanced by the incorporation of cholesterol. Positively charged molecules such as stearylamine or aminomannose or aminomannitol derivatives of cholesterol or negatively charged molecules such as dicetyl phosphate may also be incorporated into the vesicles.

When phospholipid micelles are introduced into the blood stream, the micelles move to the specific locations of cancerous growth in the patient's body. To enhance movement of the phospholipid vesicles to the specific locations, positively charged phospholipid vesicles may first be introduced into the patient's blood stream to block the macrophages or other phagocytic cells in the patient's body. The positively charged molecules bound to such phospholipid vesicles may be an aminomannose or aminomannitol derivative of cholesterol. Concurrently or after a suitable period of time such as approximately one (1) hour, other phospholipid vesicles may be introduced into the patient's blood stream to move to the specific locations in the body. Such phospholipid vesicles may include cholesterol and may be neutral or may be positively charged as by the inclusion of a stearylamine or aminomannose or aminomannitol derivative of cholesterol or may be negatively charged as by the inclusion of a dicetyl phosphate.

When the phospholipid vesicles are introduced into the body to target tumors, indium-111 may be used as the labelling agent. The indium-111 may be chelated to a suitable material such as nitrilotriacetic acid (NTA). NTA is advantageous because it forms a relatively weak bond with the indium-111. As a result, when the phospholipid vesicles reach the tumor and are lysed, the nitrilotriacetic acid is displaced by proteins at the tumor. Since the proteins form a stronger bond with indium-111, the indium-111 remains at the tumor for a long period of time (in excess of 24 hours), which provides for easy identification of the tumor over the extended period of time.

Materials and Methods

Liposome Preparation. Small unilamellar vesicles (SUV) with the ionophore A23187 were prepared from distearoyl phosphatidycholine (DSPC), cholesterol (Ch), dicetyl phosphate (DP), stearylamine (SA) and the 6-aminomannose (AM), and 6-aminomannitol (AML) derivatives of cholesterol, according to previous methods. Briefly, chloroform solutions of 10 mg lipid with the following molar ratios: DSPC:Ch, 2:1; DSPC:Ch:X, 4:1:1 where X = SA, DC or AML; and DSPC:Ch:AM, 8:3:1, were evaporated to dryness under nitrogen (N$_2$), and further dried under vacuum overnight. Each tube was filled with 0.6 ml 5mM phosphate buffered 0.9% saline, pH 7.4(PBS), containing 1mM nitrilotriacetic acid (NTA) and sonicated under N$_2$, for 5 to 15 minutes with a sonicator equipped with a titanium microtip.

Liposomes were annealed at 60°C for 10 minutes and centrifuged at 300 x g for five to ten minutes. Liposomes were separated from unencapsulated NTA with a 30 x 1.5 cm Sephadex® G-50 column. Liposome size was determined by laser light scattering. All vesicle types were shown by laser light scattering microscopy to have a mean diameter less than 0.1 nm (100 nm). For example, DSPC:Ch vesicles had a mean diameter of 52,8 nm. However, vesicles as large as approximately 200 nm are believed to be satisfactory in obtaining the desired results of this invention, although the preferred range is approximately 50 to about 70 nm.

The vesicles obtained as described above are chemically pure. By "chemically pure" is meant that the materials which constitute phospholipid vesicles are more than 98% pure. For example, when the phospholipid chemical added is distearoyl phosphatidylcholine, this material is used at more than 98% purity. The same constraint holds for other components, such as cholesterol, which compose the vesicle. The phospholipid vesicles obtained as described above are stable when injected into experimental animals.

The aminomannose and aminomannitol portions of these derivatives of cholesterol extend externally from the phospholipid particles. Thus, when such derivatives are incorporated or associated into the surfaces of vesicles or other micelles, an amine moiety is provided that extends approximately 0.5-1,5 nm preferably about 1 nm, beyond the surface of the micelles. In the case of vesicles, it appears that the appropriate molecular design comprises a hydrophobic portion which serves to anchor the molecule within the vesicular bilayer, and a linking portion which is at least mildly hydrophilic which spans the requisite distance between the hydrophobic region and the amino functional group. The hydrophilicity is apparently required to prevent the link from internalizing within the bilayer also and thus serves to "extend" the amine from the surface. An example of a successful extended amine within the context of this invention is a 6-aminomannose cholesterol derivative such as, for example, 6-(5-cholesten-3-$\beta$-yloxy)hexyl-6-amino-6-deoxyl-1-thio-$\alpha$D-mannopyranoside. In this example, the cholesterol portion provides the hydrophobic moiety, while the aminomannose is relatively hydrophilic. Other embodiments are also possible: other amino sugars attached to other cholesterol derivatives, for example, are equally suitable as alternative embodiments of the hydrophilic and hydrophobic portions. Polyamines and polyamino acids which can be bound covalently or associated by other means to the vesicle or other micelle surface may also be used.

The amino derivatives and cholesterol tend to impart stability to the phospholipid vesicles. Cholesterol may be included in the range of approximately 0% to 50% of cholesterol by weight and the remainder constituting the phospholipids. The charged molecules such as the stearylamine, the dicetyl phosphate and the aminomannose and aminomannitol derivatives of cholesterol may be included in the range of 0% to 20% by weight of the charged molecules and the remainder constituting the phospholipids.

The chemically pure liposome compositions discussed above are quite stable to leakage in vitro and in vivo. However, phospholipid mixtures such as egg lecithin form more fluid membranes than pure phospholipids. As a result, liposomes from natural lecithin mixtures are less stable to leakage of their contents than pure phopholipids.

In-111 Loading Procedure. Loading of In-111 into reformed liposomes was facilitated by the presence of A23187 in the lipid bilayer. In-111 was loaded into liposomes at 60-80°C in accordance with the procedure described by Mauh and Gamble, Anal. Biochem. 94, 302-307 (1979). Incubations were terminated by the addition of 10mM ethylenediaminetetraacetic acid (EDTA) in 10 mM phosphate buffered 0.9% sodium chloride, pH 7.4 (PBS), and free In-111 was separated from the loaded liposomes by chromatography on Sephadex G-50. Up to 90% of the added In-111 could be incorporated into preformed liposomes by this technique, and specific activities of up to 11.1 MBq/mg (300 $\mu$Ci/mg) lipid have been obtained.

All patients diagnosed by the process of this invention had biopsy-proven malignant disease diagnosed as incurable and a life-expectancy of less than two years. Patients received a dose of 18.5 MBq (500 $\mu$Ci)

of In-111 in varying amounts of vesicles (45-275 mg) such that the relationship between kinetics of distribution and clearance to lipid dose could be determined. The vesicles were of the following formulation:

| Per 100 mg lipid | mg |
|---|---|
| L-α-distearoyl/phosphatidylcholine (DSPC) | 80.70 |
| Cholesterol | 19.30 |
| Nitrilotriacetic Acid (trisodium salt) | 0.03 |
| $In-111Cl_3$ MBq (μCi) | 2.5-37 (250 - 1000 See Table 1) |
| Ionophore A23187 | 0.10 |

Patients were tested at the dosage levels indicated in Table 1. Three patients were admitted to each dose level until dose level three was attained. If toxicity was observed in any of the dose levels prior to level three, eight additional patients were to be entered at that dose level to determine adverse reaction frequency.

Twenty-four and forty-eight hours following the intravenous administration of vesicles over a three minute period, whole body and regional imaging was performed utilizing gamma camera-dedicated computer systems. Window settings were adjusted to include both 172 KeV and 247 KeV energy peaks for In-111 emissions.

TABLE 1

| DOSAGE LEVEL | | |
|---|---|---|
| LEVEL | LIPID DOSE | 111 In DOSE |
| 1 | 50 mg | 18,5 MBq (500 μCi) |
| 2 | 100 mg | 18,5 MBq (500 μCi) |
| 3 | 200 mg | 18,5 MBq (500 μCi) |
| 4 | 200 mg | 27,6 MBq (750 μCi)* |
| 5 | 200 mg | 37 MBq (1000 μCi)* |

*dosage level not used to date

Tests were performed according to the schedule in Table 2.

## TABLE 2

## PARAMETERS FOLLOWED

| | Prein- jection | 1 Hours | 4 Hours | 8 Hours | 24 Hours | 48 Hours | 72 Hours -- |
|---|---|---|---|---|---|---|---|
| Examination | X | | X | | X | X | |
| Scan | | | X (optional) | | X | X | X (optional) |
| CBC,Differential | X | | | | | X | |
| Platelet Count | X | | | | | X | |
| Chemistry-Profile and electrolytes (SMA 18) | X | | | | | X | |
| Chest X-Ray | X | | | | | X | |
| Serum Complement | X | | | | | X | |
| Urinalysis | X | | | | | X | |
| Blood Sample for Radioactivity | X | X | X | | X | X | X |
| Urine Sample for Radioactivity | X | | X | X | | | X |
| Stool Sample for Radioactivity | | | | | | X | |

RESULTS

A. DESCRIPTION OF PATIENTS/DIAGNOSES

Thirteen patients (nine men and four women) were treated and the results were analyzed at the time of this report. Their ages ranged between 39-80. Patients were diagnosed as having primary cancers of the following sites:

| Site | No. of Patients |
|------|------|
| Lung | 3 |
| Breast | 3 |
| Prostate | 3 |
| Colon | 1 |
| Pancreas | 1 |
| Kidney | 1 |
| Lymphoma | 1 |

All patients except two (prostate cancer and oat cell lung cancer) had prior treatment for their cancer: ablative surgery, radiation therapy, chemotherapy and hormonal therapy were variously used to treat specific patients. Certain patients presented signs and symptoms that were known or suspected to be secondary to their cancers (e.g. bone pain and anemia).

B. DOSAGE DATA

Patients received intravenous injections of vesicles containing 45-275 mg of lipid and in all cases, 18.5 MBq (500 microcuries) of indium-111. For kinetic studies, blood, urine and stool samples were collected over the first three days. Whole body and regional gamma camera images are obtained at 1-48 hours.

C. SAFETY DATA

None of the 13 patients developed symptoms within 72 hours after administration of the vesicles which were judged to be attributable to the test article. Patient #2 complained of weakness at 48 hours, having been recently placed in a new analgesic, Vistaril. Patient #3 complained of dizziness lasting 30 minutes which developed 8 hours post-injection. A neurological exam performed at 24 hours was within normal limits. The same patient's eosinophil count rose from 1% at baseline to 7% at 48 hours. Patient #6 developed a two degree increase in temperature and an increase in pulse (T 97.4, P 64 at baseline; T 99.9, P 90 at 8 hours). The temperature rise began at four hours and continued through 72 hours. The surgical insertion of Hickman catherter was judged to be the likely etiology. At 48 hours the same patient creatinine showed a slight increase from 0.7 mg/dl. to 0.9 mg/dl. Patient #9 showed an increase in glucose from baseline of 135 mg/dl to 194 mg/dl at 24 hours. Patient #13 showed an increase in eosinophils from 0% at baseline to 5% at 48 hours.

Otherwise, vital signs and physical examination, as well as blood and urine tests showed no change over the 72 hours following administration of the vesicles. Chest X-rays revealed no evidence of altered aeration or vascularity within the lungs.

D. RADIOPHARMACOKINETIC RESULTS

Radiokinetic determinations indicate that the liposomes were rapidly taken up by the liver and spleen. Within the first one to four hours, there was a large amount of radioactivity remaining within the blood stream as well, but by 24 hours after injection, a significant amount of vesicles had left the blood stream. A median of 12.5% of the injected vesicles remained within the circulation at 24 hours. Urinary excretion was quite small (median 0.95%) and fecal excretion was insignificantly small.

The radiation exposure to the whole body was $0.29 \times 10^{-2}$J/kg (0.29 rads) median. The patient with the highest amount of radiation exposure had only $0.30 \times 10^{-2}$J/kg (0.30 rads) whole body radiation. The dose limiting radiation exposures were liver and spleen. Usually liver exposure was slightly higher (7 patients) although in 5 patients, spleen exposure was slightly higher. The median exposure to the liver was $2.3 \times 10^{-2}$J/kg (2.3 rads) with the greatest exposure being $4.7 \times 10^{-2}$J/kg (4.7 rads) and the median exposure to the spleen was $1.6 \times 10^{-2}$J/kg (1.6 rads) with the highest exposure being $4.8 \times 10^{-2}$J/kg (4.8 rads).

At 1-4 hours after administration, images demonstrated a vascular pool. At 24 and 48 hours, the liver and spleen had accumulated significant amounts of radioactivity, and the vasculate pool was minimal (exception patient #4 where the vascular pool remained high even at 48 hours.)

E. RESULTS AND IMAGING EFFICACY

7

Twelve of the thirteen patients had tumors imaged by this scan, including three patients with breast cancer, three patients with oat cell carcinoma of the lung, three patients with prostatic carcinoma, one patient with carcinoma of the rectum, one patient with carcinoma of the kidney, and one patient with malignant lymphoma. Only in the patient with carcinoma of the pancreas was there no tumor image seen, and in that patient, the size of the tumors on the peritoneal surface was less than 5 mm.

Accumulating all of the organs which had tumor demonstated by standard techniques, 22 such organ sites were involved the tumor. Of those, 20 had tumors imaged by scans (false positive rate = 9%). The patient with carcinoma of the pancreas did not have peritoneal soft tissue tumors identified, and a second patient with oat cell carcinoma of the lung metastatic to brain which had previously been heavily irradiated had no evidence of brain metastasis on liposome scan.

In the 82 instances of organ systems that were not clinically involved with tumor by standard techniques, there was 1 instance of imaging (of bone) by liposome scan (false positive rate = 1.2%). The accuracy rate for vescan was 101/104 or 97%.

Organs which were imaged successfully by the vesicle scan included bone, lymph node, soft tissue including mediastinum, lung, liver, and spinal cord. A single patient with a brain tumor previously treated with radiation therapy was not able to be imaged.

Four cases of unsuspected tumors were observed. In patients with oat cell carcinoma, one patient with an unsuspected meningeal metastasis was identified, and another patient with an unsuspected liver metastasis was identified. One patient with carcinoma of the breast showed involvement diffusely over the right chest, and subsequently developed a malignant pleural effusion which was not present at the time of the vesicle scan. One patient with carcinoma of the prostate had a heterogeneous uptake of liposomes in the liver, and subsequently developed malignant hepatomegaly.

The results described above are summarized in Table 3 which follows:

TABLE 3

| Ptl. | Disease | Prior* Treatment | Dose Lipid | Dose In-111 | % Blood in 4h | % Blood in 24h | % Exc. Urine | Radiation Exp. Liver | Spleen | Whole Body | Bone Marrow | Detection Bone Marrow | Bone | Node | Soft Tissue | Lung | Liver | Brain | Spinal Cord | Comments | Autopsy Conf. | Toxicat. |
|---|---|---|---|---|---|---|---|---|---|---|---|---|---|---|---|---|---|---|---|---|---|---|
| 01 | Prostate | R,C,H | 62.6 | 0.5 | 65.7 | 28.5 | 1.4 | 2.0 | 0.6 | 0.27 | 0.31 | TP | TN | TN | TN | TN | TP | TN | | | 0 | 0 |
| 02 | Pancreas | R,C | 44.5 | 0.5 | 15.0 | 1.3 | 0.7 | 4.7 | 3.1 | 0.30 | 0.22 | TN | TN | TN | FN | TN | TN | TN | | CT scan false + in Pancreas | + | 0 |
| 03 | Breast | C,H | 56.8 | 0.5 | 40.4 | 12.5 | 1.2 | 2.5 | 2.2 | 0.28 | 0.28 | TN | TN | TP | TP | TP | TN | TN | | Pleural effusion 1 month later | - | 0 |
| 04 | Breast | C | 82.1 | 0.5 | 86 | 64 | 0.9 | 0.8 | 1.2 | 0.28 | 0.34 | TP | TN | TN | TP | TN | TN | TN | | | - | 0 |
| 05 | Lung, Oat Cell | R,C | 80 | 0.5 | 67.6 | 31.7 | 3.0 | 1.5 | 1.6 | 0.30 | 0.30 | TN | TP | TN | TN | TP | TP | FN | | | + | 0 |
| 06 | Breast | R,H | 98.9 | 0.5 | 42 | 12.2 | 1.0 | 2.8 | 3.1 | 0.29 | 0.28 | TP | TN | TN | TN | TN | TN | TN | | | - | 0 |
| 07 | Colon | R,C | 135 | 0.5 | 11.8 | 6.4 | 0.6 | 3.4 | 4.0 | 0.30 | 0.16 | TN | TN | TN | TP | TN | TN | TN | | | + | 0 |
| 08 | Prostate | H | 102 | 0.5 | 50.6 | 27.1 | 0.6 | 1.4 | 1.4 | 0.30 | 0.32 | TP | TN | TN | TN | TP | TN | TN | | | - | 0 |
| 09 | Kidney | C,H | 273 | 0.5 | — | 22.4 | 1.0 | 2.2 | 1.5 | 0.29 | 0.36 | TN | TN | TN | TP | TP | TN | TN | | | - | 0 |
| 10 | Lung, Oat Cell | R,C | 200 | 0.5 | 51 | 5.5 | — | 2.1 | 1.5 | 0.20 | 0.19 | TP | TN | TN | TP | TP | TN | TN | TP | | + | 0 |
| 11 | Prostate | 0 | 173 | 0.53 | ND | 9.6 | 0.2 | 2.3 | 1.1 | 0.29 | 0.29 | TP | TN | TN | TN | TN | TN | TN | | | - | 0 |
| 12 | Lung, Oat Cell | 0 | 276 | 0.5 | 5.9 | 2.0 | 2.0 | 3.8 | 1.8 | 0.29 | 0.25 | TP | TN | TN | TP | TP | TN | TN | | | - | 0 |
| 13 | Lymphoma | 0 | 200 | 0.5 | ND | 14.4 | 0.7 | 2.4 | 4.4 | 0.30 | 0.28 | FP | TP | TP | TN | TN | TN | TN | | | - | 0 |

** 

| | |
|---|---|
| TP | = True Positive |
| TN | = True Negative |
| FP | = False Positive |
| FN | = False Negative |

\*  S = Surgical Removal of Tumor
   R = Radiotherapy
   C = Chemotherapy
   H = Hormone Therapy
   O = No Prior Therapy

As indicated above, the patients were given intravenous injections of up to approximately 275 milligrams of lipid and 18.5 MBq (500 microcuries) of Indium-111. Other dose levels, however, are within the scope of the invention. Thus, the lipid dose may vary from approximately 40 milligrams to about 1 gram, with a range of about 100 to about 700 milligrams being preferred, and from about 200 to approximately 500 milligrams being especially preferred. The particular dose level of lipid will be determined on a case by case basis, with the amount being sufficient to present enough vesicles for tumor targeting and at the same time kept to as small an amount as reasonably possible since the vesicles constitute a foreign object in the human body.

In the examples herein reported, the dose of radio-labelled substance was 18.5 MBq (500 microcuries) of Indium-111. It is to be understood, however, that other dose levels and other radiolabelled substances may be utilized. Thus, for example, radiolabelled materials such as gallium 67 (Ga-67), technetium 99m (Tc-99m), and iodine 131 (I-131), may be utilized for imaging. It should also be understood that the particular dose level of radiolabelled substance will vary depending upon the specific substance utilized, as well as upon the preliminary diagnoses of the condition of the patient. Accordingly, with Indium-111, the dose level will typically range between approximately 18.5 to about 74 MBq (0.5 to about 2.0 millicuries), whereas with gallium 67 and iodine 131, the dose will ordinarily be between about 74 and approximately 185 MBq (2 and approximately 5 millicuries). With technetium 99m, however, which is known to be excreted much more readily and extensively than other radioactive elements, the dose will vary from approximately 185 to about 740 MBq (5 to about 20 millicuries).

It will also be clear that the method of this invention, in addition to targeting tumors as described, is applicable in determining whether particles designed for therapy would move to a specific tumor site for a given patient. Moreover, it has been found that the method of this invention is particularly advantageous in that it does not image arthritis or inflammations as do other imaging techniques, and that only active tumor cells are imaged rather than cells that have been treated as by radiation or chemotherapy.

## Claims

1. The use of micellular particles of less than 200 nm comprising chemically pure phospholipid molecules, said particles having incorporated a radioactive agent, for the preparation of pharmaceutical compositions for the diagnosis or treatment of tumors in humans, especially breast cancer, oat cell carcinoma, prostatic carcinoma, renal cell carcinoma, colon carcinoma or malignant lymphoma.

2. The use of micellular particles of less than 200 nm comprising chemically pure phospholipid molecules, said particles having incorporated a radioactive agent for the preparation of pharmaceutical compositions for imaging or treatment of tumors in bone, lymph node and soft tissue including mediastinum, lung, liver and spinal cord.

3. The use of micellular particles of less than 200 nm comprising chemically pure phospholipid molecules, said particles having incorporated a radioactive agent for the preparation of pharmaceutical compositions for the diagnosis or treatment of metastatic cancers arising from primary breast cancer, oat cell carcinoma, prostatic carcinoma, renal cell carcinoma or colon carcinoma in humans.

4. The use according to claims 1 to 3 wherein the radioactive agent emits gamma radiation.

5. The use according to claim 4 wherein the radioactive agent comprises Indium-111.

6. The use according to claims 1 to 3 wherein the radioactive element is Gallium 67, Technetium 99m or Iodine 131.

7. The use according to claims 1 to 3 wherein said phospholipid molecules constitute distearoyl phosphatidyl choline.

8. The use according to claim 7 wherein cholesterol is included in said phospholipid molecules to enhance the stability of said micellular particles.

9. The use according to claims 1 to 3 wherein charged molecules are attached to said micellular particles.

10. The use according to claim 7 wherein said small, chemically pure phospholipid molecules are neutral and the micellular particles further contain positively or negatively charged molecules.

11. The use according to claims 1 to 3 wherein the micellular particle composition is composed of
    (a) a first group of micellular particles comprising chemically pure phospholipid molecules, having positively or negatively charged molecules extending externally from the particles, and

10

(b) a second group of neutral micellular particles of less than 200 nm, comprising chemically pure phospholipid molecules, said particles having incorporated a radioactive agent.

12. The use according to claims 1 to 3 and 11 wherein said micellular particles provide a lipid dose level of between 40 mg to 275 mg.

13. The use according to one or more of the preceding claims wherein said micellular particles are less than 100 nm.

14. The use according to one or more of the preceding claims wherein said micellular particles are from 50 to 70 nm.

15. The use according to one or more of the preceding claims wherein said micellular particles are vesicles.

**Revendications**

1. Utilisation de particules micellulaires de moins de 200 nm comprenant des molécules de phospholipides chimiquement pures, ces particules ayant un agent radioactif incorporé, pour la préparation de compositions pharmaceutiques pour le diagnostic ou le traitement de tumeurs chez l'homme, en particulier le cancer du sein, le carcinome à cellules en grains d'avoine, le cancer de la prostate, le cancer des cellules rénales, le cancer colique ou les lymphomes malins.

2. Utilisation de particules micellulaires de moins de 200 nm comprenant des molécules de phospholipides chimiquement pures, ces particules ayant un agent radioactif incorporé, pour la préparation de compositions pharmaceutiques pour l'imagerie ou le traitement de tumeurs osseuses, des ganglions lymphatiques et des tissus mous, en particulier le médiastin, le poumon, le foie et la moelle épinière.

3. Utilisation de particules micellulaires de moins de 200 nm comprenant des molécules de phospholipides chimiquement pures, ces particules ayant un agent radioactif incorporé, pour la préparation de compositions pharmaceutiques pour le diagnostic ou le traitement de cancers métastatiques provenant d'un cancer primitif du sein, d'un carcinome à cellules en grain d'avoine, d'un cancer de la prostate, d'un cancer des cellules rénales ou d'un cancer du colon chez l'homme.

4. Utilisation selon les revendications 1 à 3 dans laquelle l'agent radioactif émet une radiation gamma.

5. utilisation selon la revendication 4 dans laquelle l'agent radioactif comprend de l'Indium-111.

6. Utilisation selon les revendications 1 à 3 dans laquelle l'élément radioactif est le Gallium 67, le Technicium 99m ou l'Iode 131.

7. Utilisation selon les revendications 1 à 3 dans laquelle les molécules de phospholipides constituent la distéaroyl phosphatidyl choline.

8. Utilisation selon la revendication 7 dans laquelle du cholestérol est inclus dans ces molécules de phospholipides pour augmenter la stabilité de ces particules micellulaires.

9. Utilisation selon les revendications 1 à 3 dans laquelle des molécules chargées sont liées à ces particules micellulaires.

10. Utilisation selon la revendication 7 dans laquelle ces petites molécules de phospholipides chimiquement pures sont neutres et les particules micellulaires contiennent en plus des molécules chargées positivement ou négativement.

11. Utilisation selon les revendications 1 à 3 dans laquelle la composition de la particule micellulaire est composée de
(a) un premier groupe de particules micellulaires comprenant des molécules de phospholipides chimiquement pures, ayant des molécules chargées positivement ou négativement s'étendant vers l'extérieur à partir des particules et

(b) un second groupe de particules micellulaires comprenant des molécules de phospholipides chimiquement pures, ces particules ayant un agent radioactif incorporé.

**12.** Utilisation selon les revendications 1 à 3 et 11 dans laquelle ces particules micellulaires fournissent un niveau de dose de lipides compris entre 40 mg et 275 mg.

**13.** Utilisation selon l'une ou plusieurs des revendications précédentes dans laquelle ces particules micellulaires ont moins de 100 nm.

**14.** utilisation selon l'une ou plusieurs des revendications précédentes dans laquelle ces particules micellulaires sont comprises entre 50 et 70 nm.

**15.** Utilisation selon l'une ou plusieurs des revendications précédentes dans laquelle ces particules micellulaires sont des vésicules.

**Patentansprüche**

**1.** Verwendung mizellärer Partikel von weniger als 200 nm, umfassend chemisch reine Phospholipidmoleküle, wobei in den Partikeln ein radioaktives Mittel eingeschlossen ist, zur Herstellung von pharmazeutischen Zusammensetzungen für die Diagnose oder Behandlung von Tumoren beim Menschen, insbesondre Brustkrebs, Haferkornzellenkarzinom, Prostatakarzinom, Nierenzellenkarzinom, Colonkarzinom oder bösartiges Lymphom.

**2.** Verwendung von mizellären Partikeln von weniger als 200 nm, umfassend chemisch reine Phospholipidmoleküle, wobei in den Partikeln ein rdioaktives Mittel eingeschlossen ist, zur Herstellung von pharmazeutischen Zusammensetzungen zur Abbildung oder Behandlung von Tumoren in Knochen, Lymphknoten und Weichgewebe, einschliesslich Mediastinum, Lunge, Leber und Rückenmark.

**3.** Verwendung mizellärer Partikel von weniger als 200 nm, umfassend chemisch reine Phospholipidmoleküle, wobei in den Partikeln ein radioaktives Mittel eingeschlossen ist, zur Herstellung von pharmazeutischen Zusammensetzungen zur Diagnose oder Behandlung von Metastasen, die aus primärem Brustkrebs, Haferkornzellenkarzinom, Prostatakarzinom, Nierenzellenkarzinom oder Colonkarzinom beim Menschen herrühren.

**4.** Verwendung nach den Ansprüchen 1 bis 3, worin das radioaktive Mittel Gammastrahlung aussendet.

**5.** Verwendung nach Anspruch 4, worin das radioaktive Mittel Indium-111 enthält.

**6.** Verwendung nach den Ansprüchen 1 bis 3, worin das radioaktive Element Gallium-67, Technitium-99m oder Jod-131 ist.

**7.** Verwendung nach den Ansprüchen 1 bis 3, worin das Phospholipdmolekül aus Distearoylphosphatidylcholin aufgebaut ist.

**8.** Verwendung nach Anspruch 7, worin Cholesterin in Phospholipidmolekülen zur Steigerung der Stabilität der mizellären Partikel enthalten ist.

**9.** Verwendung nach den Ansprüchen 1 bis 3, worin geladene Moleküle an die mizellären Partikel angeheftet sind.

**10.** Verwendung nach Anspruch 7, worin die kleinen chemisch reinen Phospholipidmoleküle neutral sind und die mizellären Partikel zusätzlich positiv oder negativ geladene Moleküle enthalten.

**11.** Verwendung nach den Ansprüchen 1 bis 3, worin die mizelläre Partikelzusammensetzung zusammengesetzt ist aus

(a) einer ersten Gruppe mizellärer Partikel aus chemisch reinen Phospholipidmolekülen mit positiv oder negativ geladenen Molekülen, die von der äusseren Oberfläche der Partikel herausragen, und

(b) einer zweiten Gruppe neutraler mizellärer Partikel von weniger als 200 nm aus chemisch reinen Phospholipidmolekülen, wobei in den Partikeln ein radioaktives Mittel eingeschlossen ist.

12. Verwendung nach den Ansprüchen 1 bis 3 und 11, worin die mizellären Partikel eine Dosierung zwischen 40 und 275 mg zur Verfügung stellen.

13. Verwendung nach einem oder mehreren der vorhergehenden Ansprüche, worin die mizellären Partikel kleiner als 100 nm sind.

14. Verwendung nach einem oder mehreren der vorhergehenden Ansprüche, worin die mizellären Partikel zwischen 50 und 70 nm sind.

15. Verwendung nach einem oder mehreren der vorhergehenden Ansprüche, worin die mizellären Partikel Vesikel sind.